# EUROPEAN PATENT APPLICATION

(11) **EP 2 022 519 A1**
(43) Date of publication of application: **11.02.2009**
(21) Application number: 07015754.0
(22) Date of filing: 10.08.2007
(51) Int. Cl.: A61M 5/142, A61M 5/168, G01L 7/08, G01L 9/00, G01L 19/00

(54) **Device for detecting a pressure change in the liquid path of a micro dosing apparatus**

(71) Applicant: F. Hoffmann-La Roche AG, 4070 Basel (CH); Roche Diagnostics GmbH, 68305 Mannheim (DE)
(72) Inventor: Bütikofer, Markus, 3110 Münsingen (CH); Bosshard, David, 3251 Wengi (CH); Kühni, Florian, 3400 Burgdorf (CH)
(74) Representative: Schalch, Rainer

(57) **Abstract**

The invention concerns an infusion pump for time controlled administration of insulin, with a permanent portion (4) and a disposable infusion set adapter (3). The infusion set adapter (3) in its interior provides an optical path (2) and furthermore comprises a liquid proof internal space (9), which in use forms a part of the liquid path (1) of the infusion pump. The boundary walls of the internal space (9) are at least partly formed by a diaphragm (10), which, with a central protruding area (18) thereof, depending on the liquid pressure in the liquid path (1) to a greater or lesser extent obstructs the optical path (2). The permanent portion (4) comprises a LED (11) and an optical sensor (12), which in use are optically connected via the optical path (2) of the infusion set adapter (3). The permanent portion (4) further comprises control means for analyzing the optical signal received by the sensor (12) in order to detect an occlusion in the liquid path (1) by an increased obstruction of the optical path (2) through the protruding area (18) of the diaphragm (10).

This concept has the advantage that the signal transmission is not susceptible with regard to the accuracy of the mechanical interfacing between the infusion set adapter (3) and the permanent portion (4), thus, the provision of infusion pumps with disposable infusion set adapters (3) is made possible which are simple, robust and inexpensive in use.

## Description

The present invention relates to a device for detecting a pressure change in the liquid path of a micro dosing apparatus, in particular for liquid drugs, to a micro dosing apparatus comprising such a device and to the use of the device and the micro dosing apparatus for dispensing of liquid drugs according to the preambles of the independent claims.

When micro-dosing liquid substances in the range of micro-liters, as is typical in the pharmaceutical and chemical industry and in particular in medical applications like e.g. the administration of liquid drugs to a patients body, in particular the time controlled administration of insulin, it is of utmost importance that the scheduled dosage is strictly followed and that an unintentional interruption in administration, as can be caused by an empty drug reservoir or an occlusion in the liquid path, can immediately be detected and remedied.

In the prior art, several concepts have been proposed so far, which, however in practical use are inexpedient, not sufficiently reliable or expensive.

For example, at insulin infusion pumps featuring a spindle driven discharge piston, it is known to monitor the driving torque or the supporting forces of the spindle and to draw, in case of a considerable increase of the respective parameter, the conclusion that an occlusion has occured in the liquid path. This method, however, has proven to be imprecise and unreliable since practically a lot of disturbing parameters, like e.g. the friction between the discharge piston and the surrounding walls of the fluid reservoir, cannot be sufficiently precise be determined and eliminated.

Furthermore, systems have been proposed in which the liquid pressure in the liquid path is determined in that an element, which is loaded by the fluid pressure and is displaceable or changes shape upon a change in fluid pressure, is mechanically coupled to a sensor unit which determines the pressure force or movement of the element, respectively, in order to calculate the liquid pressure in the liquid path. However, this concept has the drawback that the accuracy of the pressure measurement to a great extend depends on the quality of the mechanical interconnection between the element in contact with the liquid and the sensor unit, which, when the component containing the element in contact with the liquid is a disposable, which due to hygienic reasons is desirable, while the sensor unit due to cost considerations is designed for multiple use, in cases where moderate consumable costs are emphasized, typically is poor since the manufacturing accuracy of the disposables is limited.

Further, non-contact measuring systems have been proposed for systems with a similar concept, at which the displacement or change in shape of the element loaded by the fluid pressure is detected by reflecting a light beam at said element. However, even though there is no mechanical friction or play involved in the signal transmission between said element and the sensor unit, the exact position between the element performing the displacement or change in shape and the sensor unit is still crucial for the accuracy of the measurement, thus said accuracy to a great extend is depending on the manufacturing accuracy of the disposable part and thus, when consumable costs have to be kept low, is limited.

In US 2005/0178206 A1, a pump for feeding a liquid to a patients body is shown, wherein inside the pump unit optical signal emitters and optical signal receivers are oppositely arranged. The feeding line consisting of an elastic tube is displaced between them in a manner that an expansion or contraction of the tube, caused by a change in liquid pressure, can be determined by the extend of obstruction of the optical signal caused by the tube. This system, however, has the drawback that for an exchange of the feeding line (disposable tube) the pump housing must be opened, which is cumbersome and also exposes the sensitive optical elements to the risk of physical damage and/or dirt. Furthermore, also here the position of the elastic tube, which has to be installed by the user, relative to the optical signal emitter and receiver is crucial for the correct functioning of the device.

Hence, it is a general object of the invention to provide a device for detecting a pressure change in the liquid path of a micro dosing apparatus and a micro dosing apparatus comprising such a device, which at least partially avoids the disadvantages of the above mentioned prior art.

This object is achieved by the device and the micro dosing apparatus according to the independent claims.

In one aspect of the invention, the device for detecting a pressure change in the liquid path of a micro dosing apparatus for liquids, which preferably is an infusion pump for the administration of liquid drugs, preferably of insulin, comprises a first component and a second component.

The first component in its interior provides an optical path which is formed between an optical entry port and an optical exit port of said first component and is at least once deflected within said first component. Furthermore, the first component comprises a liquid proof internal space, which, via at least one opening, is connected or is connectable to the liquid path of a micro dosing apparatus for liquid drugs to fluidically communicate with said liquid path. The boundary walls of the internal space are at least partly formed by a transformation element, which, when in fluid communication with the liquid path, upon a pressure change in the liquid path performs a movement and/or change in shape. The transformation element and the optical path are functionally connected in such a manner that a movement and/or change in shape of the transformation element caused by a pressure change in the liquid path and thus in the internal space results in a change of the optical cross section of the optical path, i.e. of the cross section which defines the shape and size of a light beam that can pass through the optical path.

The second component comprises optical emitter means and a related optical exit port for emitting an optical signal and optical receiver means and a related optical entry port for receiving an optical signal.

Furthermore, the first component and the second component are adapted to be detachably connected with each other in such a manner that the optical exit port of the second component is optically connected to the optical entry port of the first component and the optical exit port of the first component is optically connected to the optical entry port of the second component in order to optically connect the optical emitter means with the optical receiver means via the optical path of the first component.

The second component furthermore comprises control means functionally connected with the optical receiver means for detecting a change of the optical signal received from the optical emitter means via the optical path which is caused by a change of the optical cross section of the optical path due to a change of the liquid pressure in the liquid path and thus of the liquid pressure in the internal space of the first component.

This concept according to the invention, at which the change in pressure in the liquid path, within the first component, is converted into a change of the optical cross section of the optical path and thus into a change of the cross section or intensity, respectively, of the optical signal returned from the first component to the second component, compared to the prior art has the advantage that the signal transmission is not susceptible with regard to the accuracy of the mechanical interfacing between the first and the second component and furthermore allows to have all elements belonging to the optical measuring system be fully encapsulated either in the first or in the second component of the device, thus preventing them from physical damage and/or dirt.

Thus, through the invention the provision of devices for detecting a pressure change in the liquid path of a micro dosing apparatus is made possible which are simple and robust in use and allow the component which is in communication with the liquid path to be formed by an inexpensive disposable part without compromising with regard to reliability and accuracy.

In a preferred embodiment, the device is designed in such a manner that the first component and the second component can be separated without the use of tools, wherein it is preferred that their mechanical interconnection is established by means of a bayonet joint. By means of this, a safe mechanical connection with a defined position of the components relative to each other can be achieved and connection/disconnection of these components is made easy.

In a further preferred embodiment of the device, the optical path is two times deflected within the first component, preferably in each case by 90°so that sections of the optical path at the optical entry port of the first component and at the optical exit port of the first component run parallel at a distance to each other. By this, it is possible to arrange the optical ports of the first and the second component in each case at a common, plain outer surface of the respective component with the optical path at each port being oriented perpendicular to said outer surface, which is preferred since it reduces the likeliness of the occurrence of detrimental effects caused by reflection and/or refraction in the interfacing area between the units.

In the preceding embodiment it is preferred that the optical entry port and the optical exit port of the first component are both provided by a one-piece optical interfacing element of a transparent material, which furthermore comprises two prisms for deflecting the optical path. By this, a simple, inexpensive design with only a few parts results, which furthermore provides the advantage that all parts defining the orientation of the optical path of the first component are by the one-piece design fixedly aligned with respect to each other and thus cannot vary between first components originating from the same production. The reflecting surfaces of the prisms may be embodied as mirror surfaces.

Furthermore, in the preceding embodiment, it is preferred that the surface of the optical interfacing element facing towards the second component is substantially plain, which promotes a simple, practical design without any recesses or niches that could be prone to soiling.

In yet a further preferred embodiment of the device, the optical cross section of the optical path of the first component at any pressure has a rectangular shape, thus the area of the optical cross section changes linearly with a displacement or change in shape of the transformation element causing an obstruction of said cross section.

In yet a further preferred embodiment of the device, the optical entry port and the optical exit port of the second component are both provided by a one-piece optical interfacing element of a transparent material, resulting in a simple, inexpensive design with only a few parts. In that case it is furthermore preferred that the surface of this optical interfacing element, which is facing towards the first component, is substantially plain. As already mentioned earlier, such a surface promotes a simple, practical design without any recesses or niches that could be prone to soiling.

In yet a further preferred embodiment of the device, the optical receiver means of the second component comprise a sensor by which the intensity and/or size of the optical beam emitted by the optical exit port of the first component that is entering the optical entry port of the second component can be determined. By this, it is quite simple to detect and quantify a pressure change in the liquid path through a detection of the change in the intensity or size of said optical beam. It is however, also envisaged to use optical receiver means which can merely detect if there is a signal or no signal, thus, can merely detect the exceeding of a certain critical pressure level.

In yet a further preferred embodiment of the device, the optical emitter means of the second component comprise a LED and the optical receiver means of the same component comprise a photodiode sensitive to the light emitted by the LED. Such elements are inexpensive, commercially available and excellently suitable for this purpose.

In yet a further preferred embodiment of the device, the internal space of the first component in use forms a part of the liquid path or a part of a bypass line to the liquid path, thus is flushed by the liquid when liquid is forwarded via the liquid path.

In another preferred embodiment, the internal space forms a blind volume communicating with the liquid path, thus is not flushed when liquid is forwarded via the liquid path.

Depending on the specific design, one of these two alternative embodiments might be more advantageous in particular with regard to the dead volume of the device, to hygienic aspects and/or to the hydraulic properties of the device.

In a further preferred embodiment of the device, the transformation element is a diaphragm, preferably of circular shape, which upon a pressure change in the internal space performs a change in shape, in particular bulges or changes its extent of bulging, respectively. Such transformation elements have the advantage that, by design, they are practically free of friction, are liquid proof and can be designed as a simple one-piece structure. Furthermore, diaphragms are, even if narrowly tolerated with respect to their physical properties, relative inexpensive when produced at industrial scale and their characteristics can be adapted in a broad range to the respective application by choosing a specific geometry (shape, thickness) and a specific material (elasticity), e.g. for achieving a maximal sensitivity at a minimum change in volume. The narrow tolerances achievable with diaphragms furthermore provide the advantage that from diaphragm to diaphragm the differences in the shape changing characteristics are very small and therefore the deviations in response characteristics of first components formed with diaphragms originating from the same production can be neglected. Any remaining individual "offset" of the first components that might be caused by manufacturing and positioning tolerances can easily be eliminated through zero compensation or relative measurement, respectively.

In the preceding embodiment it is furthermore preferred that the diaphragm has a three-dimensional, preferably rotationally symmetrical shape, which allows an individual adaptation to the respective use.

Furthermore, it is preferred that it comprises a circumferential corrugation and/or a central protruding area, in the first case resulting in a soft response characteristics, i.e. a relative large change in shape takes place at moderate pressure changes, and in the second case enabling the use of the protruding area of the transformation element for obstructing the optical path, thus there is no need for additional elements for serving this purpose.

It is furthermore preferred that the diaphragm is made of a metal or a metal alloy, in particular of Titanium or an alloy thereof, or is made of plastics, in particular of PET. These materials have proven especially suitable for forming diaphragms with suitable properties.

In another preferred embodiment of the device, the transformation element is an elastic tube or an elastic blister, which upon a pressure change in the internal space expands or shrinks, preferably performs a change in diameter. Such transformation elements can be provided at low costs, e.g. by a section of a suitable semi-finished tube material.

In yet another preferred embodiment of the device, the transformation element is a rigid element, which, upon a pressure change in the internal space, performs a movement relative to other elements of the first component. Preferably, the movement is a translational displacement, a rotation or a tilting movement.

In such embodiments it is preferred that the transformation element is formed as a piston-cylinder combination, wherein the piston, which is sealed against the walls of the cylinder and is preferably spring supported, upon a pressure change in the internal space performs a guided displacement within the cylinder. This concept shows the advantage that the piston in all directions, except the direction in which it can move due to a pressure change, has a defined position relative to the remaining parts of the first component, thus an influence on the measurement through an undefined movement of the transformation element within the first component is not possible at these embodiments.

In yet a further preferred embodiment of the device, the transformation element is a piston or disc shaped element which is guided in a displaceable manner and sealed against the stationary parts of the first component by means of a circumferential elastic bead or corrugation, and which, upon a pressure change in the internal space, performs a displacement or tilting movement relative to other elements of the first component. This embodiment provides the advantage that it is practically frictionless and thus responds to even very small pressure changes.

In yet a further preferred embodiment of the device, the transformation element in a state, in which the internal space is at zero pressure, at its side facing the internal space abuts onto a contact surface, so that in the non-pressurized state it has a defined position. Preferably, this contact surface comprises a surface pattern, which by advantage shows notches, ridges or pyramids. By this it can be ensured that in case of a sudden pressure increase the entire surface of the transformation element is immediately pressurized and the risk that it sticks to the contact surface is considerably reduced. In this context, it is also desirable to furnish at least the contact surface or the entire surface of the internal space with an anti-stick coating.

In yet a further preferred embodiment of the device, the control means are adapted to continuously, in intervals or event-driven analyse the optical signal received by the optical receiver means. It is also envisaged that, at the choice of the user, the control means can be put into the respective operation mode. While a continuous analysis from a technical point of view can be considered the ideal case, which however requires a corresponding power supply and therefore in most cases will only be favoured in stationary applications, performing the analysis in intervals or event-driven is of particular advantage for battery powered mobile devices, since in these operation modes, the battery life can be considerably extended with only little compromising on the detecting speed. For example, an analysis is performed only every 30 seconds and/or when liquid is expelled via the liquid path and/or shortly before and shortly after liquid is expelled. In the last mentioned case, the occurrence of a certain difference in the liquid pressures before and after the expelling of liquid could be the criteria for detecting an occlusion.

Further preferred embodiments of the device comprise control means which are adapted to compare a signal received by the optical receiver means with an in particular user-definable signal value and to issue upon exceeding or undershooting of said value an alarm. This alarm can in particular be made noticeable acoustically, optically and/or in tactile manner. By this it is possible to automatically monitor the liquid path of micro dosing apparatuses e.g. with respect to occlusions.

Furthermore, it is preferred that the control means are adapted to correct a signal received by the optical receiver means, in particular before comparing same with a target or limit value, by means of a correction algorithm with respect to certain failures, preferably with respect to the influence of the ambient pressure, of the ambient temperature or of vibrations on the signal. By this, the accuracy and reliability can further be increased.

In yet a further preferred embodiment, the device is an independent functional unit, which for monitoring the liquid path of a micro dosing apparatus can be fluidically coupled to said liquid path, which, at micro dosing apparatuses for liquid drugs, preferably insulin pumps, can be done by using standardized catheter connecting elements, e.g. by means of a standard-luer.

In an alternative preferred embodiment, the device is an integral part of a micro dosing apparatus, preferably for liquid drugs, like e.g. an insulin pump for the time controlled administration of insulin to a patient, wherein it is preferred that the second component providing the measuring and control means is firmly and non-detachably without the aid of tools connected with the housing of the micro dosing apparatus, while the first component, which in use is in fluid communication with the liquid path, is designed as a disposable part which can be exchanged without any tools.

A second aspect of the invention relates to a micro dosing apparatus, preferably for the time-controlled administration of a liquid drug, preferably of insulin. The micro dosing apparatus comprises a dosing pump, a reservoir for the liquid to be dispensed and a device according to the first aspect of the invention. This device is designed and implemented into the micro dosing apparatus in such a manner that its first component, without the aid of any tools, can be separated from its second component while the second component stays at the remaining parts of the apparatus, preferably in a manner that without the aid of tools it cannot be separated.

In a preferred embodiment of the micro dosing apparatus, the device according to the first aspect of the invention is arranged at the apparatus in such a manner that, in use, its internal space communicates with the liquid path downstream of the dosing pump. This is in particular preferred in embodiments where the dosing pump is formed by the reservoir and a motor driven piston arranged within said reservoir, as is the case with most of the insulin pumps, since in this case there is no suction side to be monitored and monitoring the pressure side of the pump during and after administration of the liquid drug can, if the system is sensitive enough, yield information regarding the dosing pump as well as regarding the liquid path. For example, if the pressure in the liquid path is monitored from shortly before the start of operation of the dosing pump until shortly after the stop of the pump, the first pressure increase in the liquid path indicates that the dosing pump is expelling liquid, thus the pump is working and the reservoir still contains liquid, and the pressure drop shortly after shut down of the dosing pump indicates that the liquid path is functioning correctly, thus does not have an occlusion.

In a further preferred embodiment of the micro dosing apparatus, the second component of the device according to the first aspect of the invention forms together with the dosing pump or at least with the drive of the dosing pump of the apparatus an integral unit and is designed to be used several times while the first component of the device according to the first aspect of the invention communicating with its internal space with the liquid path is formed by a disposable infusion set adapter or infusion set. This concept has the advantage that the expensive parts like dosing pump or dosing pump drive, respectively, controls and measuring means are all included in one multi-use unit, while the hygienically critical part, which can only be used for a very limited time, is formed by a consumable which is changed when the reservoir is empty or when the infusion set is changed. Thus, a micro dosing apparatus can be provided which combines low operational costs with a high hygienic standard.

In yet a further embodiment of the micro dosing apparatus, the fluid connection between the internal space of the first component of the device according to the first aspect of the invention and the liquid path is established via a hollow needle protruding a septum, and the mechanical connection between the first and the second component is established by a bayonet joint. In this case, the rotational center of the bayonet joint is identical to the longitudinal axis of the hollow needle, so that upon rotating the parts forming the bayonet joint relative to each other in order to establish or release said bayonet joint, the hollow needle rotates within the septum around its longitudinal axis.

In such embodiments, it is of advantage that the hollow needle and at least one locking element of the bayonet joint is arranged at a first end of the first component and at an opposite end thereof there is arranged at least one further locking element of the bayonet joint, so that upon rotation around the longitudinal axis of the hollow needle the first component and the second component can be positively connected to each other in a defined position or be separated from each other, respectively.

These constructions make it possible to establish the mechanical as well as the fluidic connection of the first component with the second component and with the remaining parts of the apparatus by a single manipulation, namely by attaching the first and the second component to each other and establishing the bayonet joint between them.

A third aspect of the invention relates to the use of the device according to the first aspect of the invention or of the micro dosing apparatus according to the second aspect of the invention for dispensing of liquid drugs, in particular of insulin.

Further preferred embodiments of the invention become apparent from the dependent claims and from the following description by way of the drawings. Therein show:
Fig. 1 a sectional view through the top portion of an infusion pump according to the invention;
Fig. 1a an illustration as in Fig. 1 of the infusion pump of Fig. 1 with the infusion set adapter separated from the pump unit;
Fig. 2 an illustration as in Fig. 1 of a second infusion pump according to the invention;
Fig. 3 an illustration as in Fig. 1 of a third infusion pump according to the invention; and
Fig. 4 an illustration as in Fig. 1 of a fourth infusion pump according to the invention.

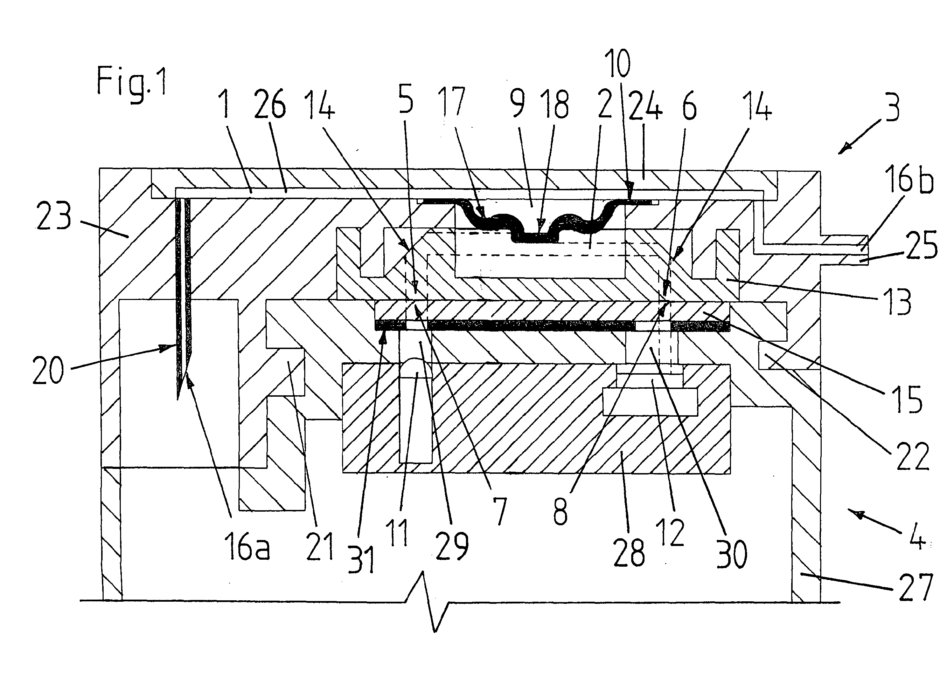

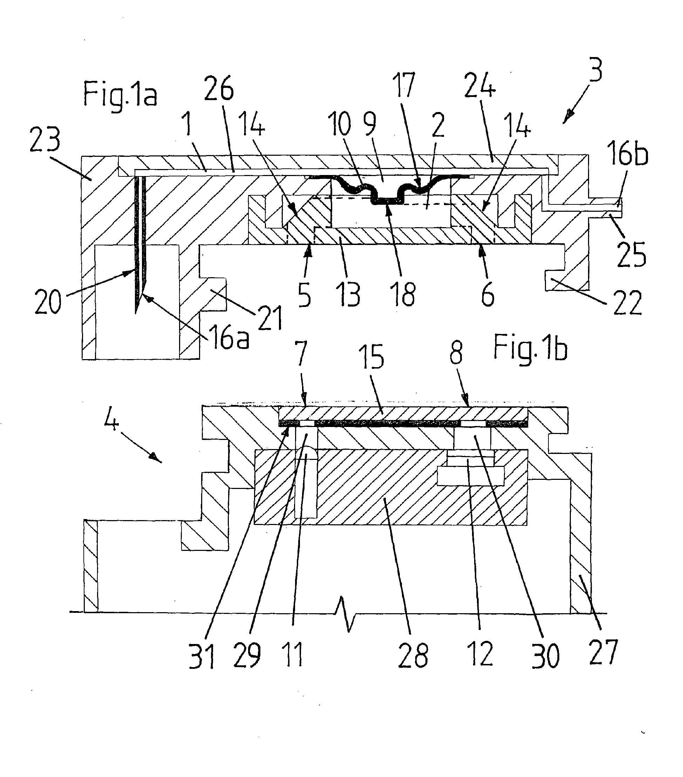

The top portion of a first preferred embodiment of the invention in the form of an infusion pump according to the second aspect of the invention for the time controlled administration of insulin is illustrated in the Figures 1 and 1a in section. Therein show Fig. 1 the top portion of the infusion pump in the assembled state and Fig. 1a said top portion in a state with the disposable infusion set adapter 3 separated from the remaining parts of the infusion pump. This disposable infusion set adapter 3 in the present embodiment forms the first component according to the claims.

As can best be seen in Fig. 1a, the disposable infusion set adapter 3 comprises a housing 23 which forms, together with a cover 24 and a diaphragm 10, which is made of a metal or plastics foil, a liquid tight internal space 9. The cover 24 and the housing 23, which are made of thermoplastic material (housing: PC/ABS (compounded blend of Polycarbonate and Acrylonitrile Butadiene Styrene), cover: MABS (Methyl Methacrylate-Butadiene-Styrene copolymer), both available from BASF), are connected with each other by ultrasonic welding, while the diaphragm 10 is glued or welded onto a flange of the housing 23.

One end of the internal space 9 is formed by a hollow needle 20 providing an entry opening 16a. The needle 20 in use protrudes through a septum provided by the insulin reservoir (not shown) of the infusion pump. Said reservoir in the present case is forming a part of the dosing pump in that it is a vial with a displacement piston which in use is driven by electrical drive means (not shown) inside the vial, thereby expelling insulin through the hollow needle 20. Thus, in use the internal space 9 of the infusion set adapter 3 is via the entry opening 16a of the hollow needle 20 fluidically connected to the exit of the dosing pump of the infusion pump.

At the other end of the internal space 9, which to a great extent is formed by a groove 26 in the cover 24, the housing 23 forms a nipple 25 providing an exit opening 16b to which the catheter (not shown) leading to the patient is attached. Thus, in the present case, the insulin to be administered is forwarded through the internal space 9 of the infusion set adapter 3, which forms part of the liquid path 1 of the infusion pump.

The cover 23 is transparent to allow visual inspection of the fluid path 1 within the infusion set adapter 3.

The diaphragm 10, which forms part of the inner walls of the internal space 9 and is liquid tight connected at its circumference to a flange of the housing 23, comprises a circumferential corrugation 17 and a central protruding area 18, which upon a pressure change in the internal space 9 moves in vertical direction upwards or downwards, depending on the change in pressure.

As can further be seen in the Figures 1 and 1a, the housing 23 at its bottom side opposite the diaphragm 10 comprises an optical interfacing element 13 of transparent material (MABS (Methyl Methacrylate-Butadiene-Styrene copolymer), available from BASF), which has a plain outer surface and on its side facing the diaphragm 10 provides two 90° prisms 14 facing each other at a distance. Between the two prisms 14 the protruding area 18 of the diaphragm 10 is arranged. By this, the optical interfacing element 13 forms an optical path 2 (see the dotted lines) within the infusion set adapter 3, which starts at an optical entry port 5 formed at the plain surface of said element 13, is then two times deflected by 90° in each case by one of the prisms 14 and ends at an optical exit port 6 which again is formed at the plain surface of the optical interfacing element 13. At the optical entry port 5 and at the optical exit port 6, the optical path 2 forms parallel section which run at a distance to each other and perpendicular to the plain surface of the optical interfacing element 13. Between the two prisms 14, the optical path runs parallel to said plain surface and to the moving direction of the protruding area 18 of the diaphragm 10. The housing 23 and the diaphragm 10 are opaque to prevent light from entering the optical path 2 at other locations than the optical ports 5, 6.

As can be seen from the dotted lines indicating the outer limits of the optical path 2, the optical path 2 is obstructed to a greater or lesser extent by the protruding area 18 of the diaphragm 10, depending on the liquid pressure in the internal space 9 which acts on the diaphragm 10. As a result, depending on the liquid pressure in the internal space 9 and in the liquid path 1, respectively, the optical cross section of the optical path 2 is more or less restricted, thus, the size of the optical path 2 varies with the liquid pressure and serves as criteria for the liquid pressure in the liquid path 1.

In the present case, the diaphragm 10 has an active diameter of 5 mm. This is the effective area where the fluid pressure effects a change in shape, stretches the diaphragm 10 and expands its central area 18 further into the optical path 2. The outer diameter of the diaphragm 10, where it is attached to the housing 23 by e.g. glueing or ultrasonic welding, is 7 mm. Upon application of a pressure of 0.5 bar, the expansion of the central protrusion 18 in direction towards the optical path 2 is in the range between 0.1 mm to 0.2 mm. The expansion strongly depends on the exact shape of the diaphragm 10 and on the elasticity (modulus of elasticity) of the diaphragm material. In the present case, the specific shape of the circumferential corrugation 17, which contributes mainly to the expansion of the diaphragm, has been optimized by the help of finite element calculations, simulations and practical tests. A suitable design of the circumferential corrugation 17 and the central protrusion 18 leads to a linear expansion characteristic of the diaphragm 10, which is preferred for its use as transformation element in the devices according to the invention.

The permanent portion 4 of the infusion pump is only partly shown in the drawings 1 and 1a, i.e. only the top portion forming the second component 4 of the device according to the first aspect of the invention is shown.

As can best be seen in Fig. 1a, it comprises a housing 27 inside which the optical emitter means 11, the optical receiver means 12 and the controls (not shown) according to the claims are arranged by means of a separate support structure 28. In the present case, the optical emitter means are formed by an infrared-LED 11 with integrated lens and the optical receiver means are formed by a photodiode 12 that is adapted to receive and convert the light emitted by the LED 11 into an electrical signal. The top side of the housing 27 comprises two openings 29, 30 to allow optical access to the LED 11 and to the photodiode 12.

The two openings 29, 30 towards the outside of the housing 27 are covered by an opaque orifice plate 31 limiting the optical cross section of the openings to a square area of 0.5 mm by 0.5 mm in each case and by a transparent cover 15 with a plain top surface, which constitutes a one-piece optical interfacing element according to the claims. At this plain surface of the cover 15, in the respective areas above the openings 29, 30, the optical exit port 7 according to the claims of the LED 11 and the optical entry port 8 according to the claims of the photodiode 12 are formed, i.e. those areas 7, 8 at which light emitted by the LED 11 can leave to the outside and at which light from the outside can enter and reach the photodiode 12.

The cover 15, the orifice plate 31 and the housing 27 are made of thermoplastic material (housing and orifice plate: PC/ABS (compounded blend of Polycarbonate and Acrylonitrile Butadiene Styrene), cover: MABS (Methyl Methacrylate-Butadiene-Styrene copolymer), both available from BASF) and are connected with each other by ultrasonic welding. The housing 27 is opaque to prevent light from entering at other locations than the optical ports 7, 8.

Instead of using an orifice plate 31, the openings 29, 30 in the housing 27 can be adapted to limit the optical cross sections in a suitable manner.

As can best be seen in Fig. 1, the infusion set adapter 3 forming the first component according to the claims and the top portion of the permanent portion 4 of the infusions pump forming the second component according to the claims are adapted to be connected with each other in such a manner that in the connected state the optical exit port 7 of the permanent portion 4 is positioned directly adjacent to the optical entry port 5 of the infusion set adapter 3, while the optical exit port 6 of the infusion set adapter 3 is positioned directly adjacent to the optical entry port 8 of the permanent portion 4, so that the LED 11 and the photodiode 12 are optically connected via the optical path 2 of the infusion set adapter 3. Depending on the position of the protruding area 18 of the diaphragm 10, which depends on the liquid pressure in the liquid path 1 and in the internal space 9, the light emitted by the LED 11 to a greater or lesser extent is received by the photodiode 12. The amount of light received is analyzed by the control means of the infusion pump and in case it falls below a preset value, an acoustic occlusion alarm is emitted.

As can further be seen from the drawings, the infusion set adapter 3 is mechanically connected with the permanent portion 4 of the infusion pump by means of a bayonet joint, which without the aid of any tools can be established and released, respectively. The rotational center of the bayonet joint falls together with the longitudinal axis of the hollow needle 20, which is arranged together with a first locking element 21 of the bayonet joint at the left end of the infusion set adapter 3, while a second locking element 22 of the bayonet joint is arranged at the right end of the infusion set adapter 3.

As becomes apparent when considering both drawings Fig. 1 and Fig. 1a, the mechanical as well as the fluidic interconnection between the permanent portion 4 of the infusion pump and the infusion set adapter 3 can be established by simply inserting the hollow needle 20 into the septum of the insulin vial (not shown) until the housings 23, 27 of the infusion set adapter 3 and of the permanent portion 4 of the infusion pump abut each other and then rotating the infusion set adapter 3 around the longitudinal axis of the needle 20 relative to the permanent portion 4 of the infusion pump until the locking elements 21, 22 positively lock with respective elements of the permanent portion 4. For removal of the infusion set adapter 3, the procedure is reversed.

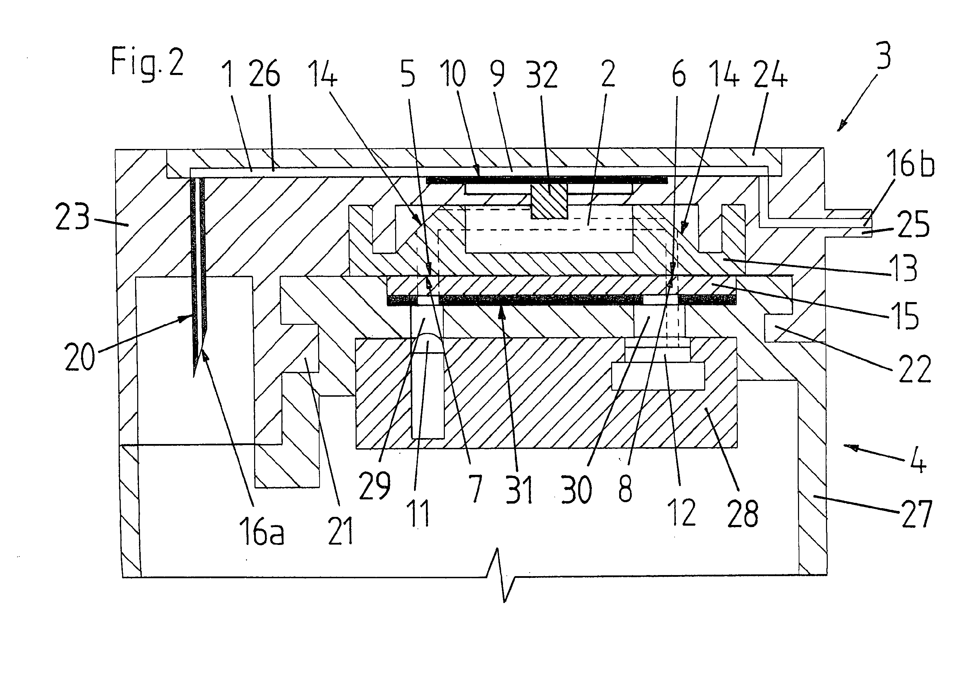

Fig. 2 shows an illustration as in Fig. 1 of a second infusion pump according to the invention, which differs from the before described in that the transformation element according to the claims is not solely formed by a diaphragm but by a combination of a diaphragm 10 and a rigid piston shaped element 32, which is connected with the centre of the diaphragm 10 and is guided in displaceable manner in an opening in the housing 23. Upon a pressure change in the internal space 9, the piston shaped element 32 is vertically moved by the diaphragm 10 and thus depending on the liquid pressure in the internal space 9, the piston shaped element 32 to a greater or lesser extent obstructs the optical path 2.

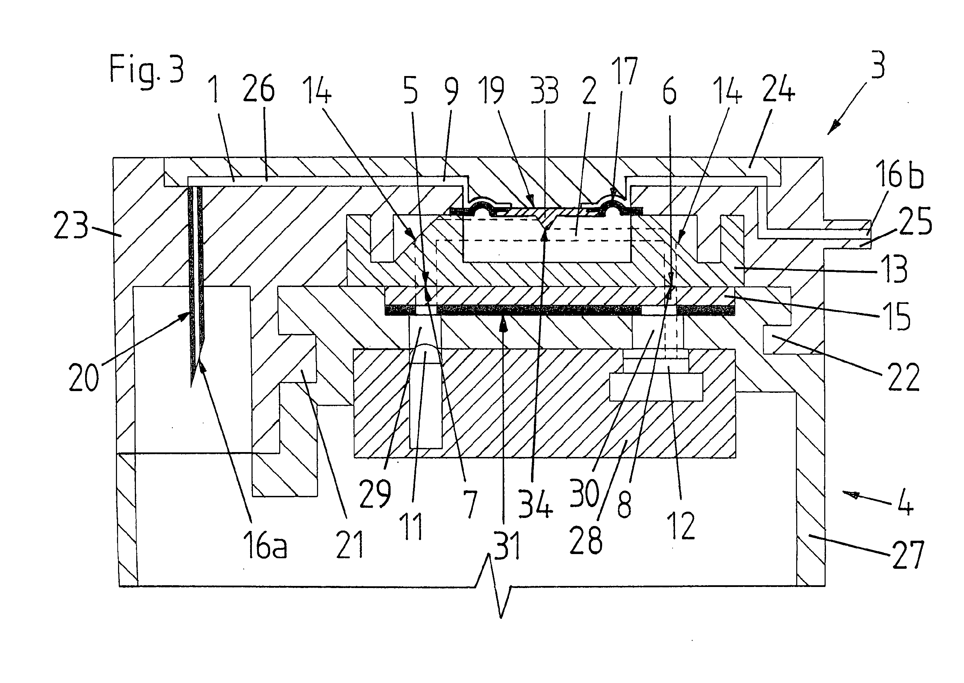

Fig. 3 shows an illustration as in Fig. 1 of a third infusion pump according to the invention, which differs from the embodiment shown in Fig. 1 in that the transformation element according to the claims is not a diaphragm but a disc shaped element 33, which is guided in displaceable manner and sealed by means of a circumferential elastic corrugation 17. The disc shaped element 33 at its side facing the optical path 2 carries a protruding ridge 34, which extends into the optical path 2. Upon a pressure change in the internal space 9, the disc shaped element 33 performs a vertical movement and, depending on the liquid pressure, with its ridge 34 to a greater or lesser extent obstructs the optical path 2. As can further be seen from Fig. 3, in the illustrated state in which the internal space 9 is at zero pressure, the disc shaped element 33 at its side facing the internal space 9 abuts onto a contact surface 19, thus in this state has a defined vertical position.

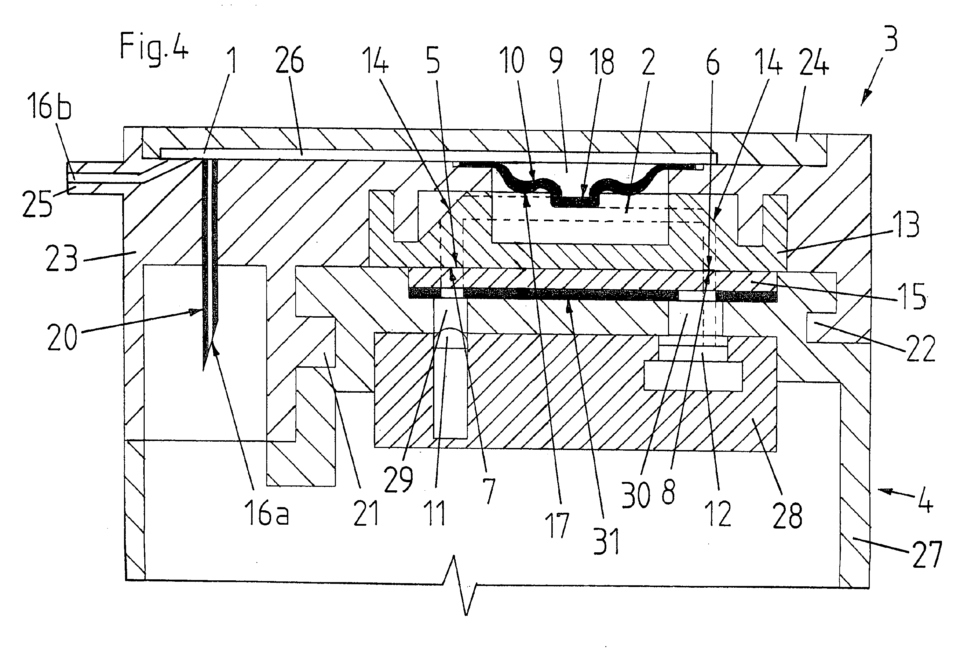

Fig. 4 shows an illustration as in Fig. 1 of a fourth infusion pump according to the invention, which differs from the embodiment shown in Fig. 1 in that the internal space 9 forms a blind volume communicating with the liquid path 1, thus is not flushed by the liquid flowing through the liquid path 1.

While there are shown and described presently preferred embodiments of the invention, it is to be distinctly understood that the invention is not limited thereto but may be otherwise variously embodied and practiced within the scope of the following claims.

## Claims

1. Device for detecting a pressure change in the liquid path (1) of a micro dosing apparatus for liquids, in particular for liquid drugs, the device comprising:
a first component (3)
having an optical path (2) formed between an optical entry port (5) and an optical exit port (6) of said first component (3), which optical path (2) is at least once deflected within said first component (3),
and having a liquid proof internal space (9), which, via at least one opening, is connected or is connectable to the liquid path (1) of a micro dosing apparatus for liquid drugs to communicate therewith, wherein at least a part of the boundary walls of the internal space (9) are formed by a transformation element (10, 18; 10, 32; 17, 33, 34) that, when the internal space (9) is connected to the liquid path (1), upon a pressure change in the liquid path (1) performs a movement and/or change in shape,
wherein the first component (3) is designed in such a manner that a movement and/or change in shape of the transformation element (10, 18; 10, 32; 17, 33, 34) caused by a pressure change in the liquid path (1) results in a change of the optical cross section of the optical path (2); and
a second component (4)
having optical emitter means (11) with a related optical exit port (7) for emitting an optical signal and optical receiver means (12) with a related optical entry port (8) for receiving an optical signal,
wherein the first component (3) and the second component (4) are adapted to be connected with each other in such a manner that the optical exit port (7) of the second component (4) is optically connected to the optical entry port (5) of the first component (3) and the optical exit port (6) of the first component (3) is optically connected to the optical entry port (8) of the second component (4) in order to optically connect the optical emitter means (11) with the optical receiver means (12) via the optical path (2) of the first component (3),
and wherein the second component (4) furthermore comprises control means functionally connected with the optical receiver means (12) for detecting a change of the optical signal received from the optical emitter means (11) via the optical path (2) caused by a change of the optical cross section of the optical path (2).

2. Device according to claim 1, wherein the device is designed in such a manner that the first (3) and the second component (4) can be separated without the use of tools, and in particular, wherein their interconnection is established by means of a bayonet joint.

3. Device according to one of the claims 1 to 2, wherein the optical path (2) inside the first component (3) is two times deflected, in particular in each case by 90°.

4. Device according to claim 3, wherein the optical entry port (5) and the optical exit port (6) of the first component (3) are both provided by a one-piece optical interfacing element (13) of a transparent material, which furthermore comprises two prisms (14) for deflecting the optical path (2).

5. Device according to claim 4, wherein the surface of the optical interfacing element (13) facing towards the second component (4) is substantially plain.

6. Device according to one of the preceding claims, wherein the optical cross section of the optical path (2) at any pressure has a rectangular shape.

7. Device according to one of the preceding claims, wherein the optical entry port (8) and the optical exit port (7) of the second component (4) are both provided by a one-piece optical interfacing element (15) of a transparent material, and in particular, wherein the surface of this optical interfacing element (15) facing towards the first component (3) is substantially plain.

8. Device according to one of the preceding claims, wherein the optical receiver means (12) comprise a sensor by which the intensity and/or size of the light beam entering the optical entry port (8) of the second component (4) can be determined.

9. Device according to one of the preceding claims, wherein the optical emitter means (11) comprise a LED and the optical receiver means (12) comprise a photodiode sensitive to the light emitted by the LED.

10. Device according to one of the preceding claims, wherein the internal space (9) in use forms a part of the liquid path (1) or a part of a bypass line to the liquid path (1).

11. Device according to one of the claims 1 to 9, wherein the internal space (9) in use forms a blind volume communicating with the liquid path (1).

12. Device according to one of the preceding claims, wherein the transformation element (10, 18; 10, 32; 17, 33, 34) is a diaphragm (10), in particular of circular shape, which upon a pressure change in the internal space (9) performs a change in shape, in particular bulges or changes its extent of bulging, respectively.

13. Device according to claim 12, wherein the diaphragm (10) has a three-dimensional, in particular rotationally symmetrical shape, and in particular comprises a circumferential corrugation (17) and/or a central protruding area (18).

14. Device according to one of the claims 12 to 13, wherein the diaphragm (10) is made of a metal or a metal alloy, in particular of Titanium or an alloy thereof, or is made of plastics, in particular of PET.

15. Device according to one of the claims 1 to 11, wherein the transformation element (10, 18; 10, 32; 17, 33, 34) is an elastic tube or an elastic blister, which upon a pressure change in the internal space (9) expands or shrinks, in particular performs a change in diameter.

16. Device according to one of the claims 1 to 11, wherein the transformation element (10, 18; 10, 32; 17, 33, 34) is a rigid element, which upon a pressure change in the internal space (9), performs a movement relative to other elements of the first component (3), in particular a displacement, a rotation or a tilting movement.

17. Device according to claim 16, wherein the transformation element (10, 18; 10, 32; 17, 33, 34) is a piston sealingly guided in a cylinder, in particular a spring supported piston, which upon a pressure change in the internal space (9) performs a displacement within the cylinder.

18. Device according to one of the claims 1 to 11, wherein the transformation element (10, 18; 10, 32; 17, 33, 34) is a piston (32) or disc shaped element (33) which is guided in displaceable manner and is sealed by means of a circumferential elastic bead or corrugation (17), and which, upon a pressure change in the internal space (9), performs a displacement or tilting movement relative to other elements of the first component (3).

19. Device according to one of the preceding claims, wherein the transformation element (10, 18; 10, 32; 17, 33, 34) in a state, in which the internal space (9) is at zero pressure, at its side facing the internal space (9) abuts onto a contact surface (19), and in particular, wherein this contact surface (19) comprises a surface pattern, in particular with notches, ridges or with pyramids.

20. Device according to one of the preceeding claims, wherein the control means are adapted to continuously, in intervals or event-driven analyze the optical signal received by the optical receiver means (12).

21. Device according to one of the preceding claims, wherein the control means are adapted to compare a signal received by the optical receiver means (12) with an in particular user-defineable signal value and to issue, upon exceeding or undershooting of said value, an alarm, which may in particular be made noticeable acoustically, optically and/or in tactile manner.

22. Device according to one of the preceding claims, wherein the control means are adapted to correct a signal received by the optical receiver means (12), in particular before comparing same with a target or limit value, by means of a correction algorithm with respect to certain failures, in particular with respect to the effect of the ambient pressure, of the ambient temperature or of vibrations on the signal.

23. Device according to one of the preceding claims, wherein the device is an independent functional unit or is integrated into a micro dosing apparatus, in particular for dispensing liquid drugs.

24. Micro dosing apparatus, in particular for the time-controlled dispensing of a liquid drug, comprising a dosing pump and a reservoir for the liquid to be dispensed, as well as a device according to one of the claims 1 to 23, wherein the first (3) and the second component (4) are designed in such a manner that the first component (3), without the aid of any tools, can be separated from the second component (4) while the second component (4) stays at the rest of the device, in particular in a manner, that without tools it cannot be separated from the rest of the device.

25. Micro dosing apparatus according to claim 24, wherein the device according to one of the claims 1 to 23 is arranged in the liquid path (1) downstream of the dosing pump.

26. Micro dosing apparatus according to one of the claims 24 to 25, wherein the second component (4) forms an integral unit together with the dosing pump and is designed to be used several times while the first component is formed by a disposable infusion set adapter (3) or an infusion set.

27. Micro dosing apparatus according to one of the claims 24 to 26, wherein the fluid connection between the internal space (9) of the first component (3) and the liquid path (1) is established via a septum and hollow needle (20) connection and the mechanical connection between the first (3) and the second component (4) is established by a bayonet joint, wherein the rotational center of the bayonet joint falls together with the longitudinal axis of the hollow needle (20).

28. Micro dosing apparatus according to claim 27, wherein the hollow needle (20) and at least one locking element (21) of the bayonet joint is arranged at a first end of the first component (3) and at an opposite end thereof there is arranged at least one further locking element (22) of the bayonet joint, so that upon rotation around the longitudinal axis of the hollow needle (20) the first component (3) and the second component (4) can be positively connected to each other in a defined position or be separated from each other, respectively.

29. Use of the device according to one of the claims 1 to 23 or of the micro dosing apparatus according to one of the claims 24 to 28 for dispensing of liquid drugs, in particular insulin.
